# EUROPÄISCHE PATENTSCHRIFT

(11) **EP 2 024 727 B1**
(45) Veröffentlichungstag und Bekanntmachung des Hinweises auf die Patenterteilung: **17.03.2010**
(21) Anmeldenummer: 07724498.6
(22) Anmeldetag: 24.04.2007
(51) Int. Cl.: G01N 15/04, G01N 33/50, G01N 33/58

(54) **VERFAHREN ZUR BESTIMMUNG DER VIABILITÄT VON ZELLEN IN ZELLKULTUREN**
METHOD FOR DETERMINING THE VIABILITY OF CELLS IN CELL CULTURES
PROCÉDÉ DE DÉTERMINATION DE LA VIABILITÉ DE CELLULES DANS DES CULTURES CELLULAIRES

(30) Priorität: 15.05.2006 DE 102006022877
(43) Veröffentlichungstag der Anmeldung: 18.02.2009
(73) Patentinhaber: Sartorius Stedim Biotech GmbH, 37079 Göttingen (DE)
(72) Erfinder: OBERMANN, Stefan, 37139 Adelebsen (DE); BAUMFALK, Reinhard, 37083 Göttingen (DE); REIF, Oscar-Werner, 30173 Hannover (DE); WURM, Florian, CH-1092 Belmont-sur-Lausanne (CH); DE JESUS, Maria, CH-1022 Chavannes (CH); STETTLER, Matthieu, CH-1095 Lutry (CH); JORDAN, Martin, CH-1024 Ecublens (CH)
(86) Internationale Anmeldenummer: PCT/EP2007/003568
(87) Internationale Veröffentlichungsnummer: WO 2007/131596

(56) Entgegenhaltungen:
- EP-A- 1 293 205
- WO-A-2005/087253
- SCHOENHERR ILONKA ET AL: "Comparison of different methods to determine the end of exponential growth in CHO cell cultures for optimization of scale-up" BIOTECHNOL PROG; BIOTECHNOLOGY PROGRESS SEP 2000 AICHE, NEW YORK, NY, USA, Bd. 16, Nr. 5, September 2000 (2000-09), Seiten 815-821, XP002439446
- SCHREER ET AL: "Application of Alamar blue/5-carboxyfluorescein diacetate acetoxymethyl ester as a noninvasive cell viability assay in primary hepatocytes from rainbow trout" ANALYTICAL BIOCHEMISTRY, ACADEMIC PRESS, NEW YORK, NY, US, Bd. 344, Nr. 1, 1. September 2005 (2005-09-01), Seiten 76-85, XP005012202 ISSN: 0003-2697
- MACEYKA M ET AL: "Aminoacylase 1 is a sphingosine kinase 1-interacting protein" FEBS LETTERS, ELSEVIER, AMSTERDAM, NL, Bd. 568, Nr. 1-3, 18. Juni 2004 (2004-06-18), Seiten 30-34, XP004516402 ISSN: 0014-5793
- LUECKE J: "Fast, reproducible and reliable determination of biomass in suspension cell cultures with VoluPAC tubes" NATURE METHODS, Oktober 2006 (2006-10), Seiten V-VI, XP009085689
- STETTLER M ET AL: "New disposable tubes for rapid and precise biomass assessment for suspension cultures of mammalian cells" BIOTECHNOLOGY AND BIOENGINEERING, Bd. 95, Nr. 6, 20. Dezember 2006 (2006-12-20), Seiten 1228-1233, XP002439286

## Beschreibung

Die Erfindung bezieht sich auf ein Verfahren zum Bestimmen der Viabilität von Zellen in Zellkulturen, umfassend die Schritte:
- Anfärben der Probe mit einem Farbstoff, der in Abhängigkeit von der Viabilität einzelner Zellen in diese einzudringen vermag,
- Bestimmen der Anteile unterschiedlich stark angefärbter Zellen.

Zur Bestimmung der Viabilität, d.h. des "Gesundheitszustandes" von Zellen in einer Zellkultur ist das so genannte Tryphanblau-Verfahren bekannt. Dabei werden Zellen in Suspension mit dem Farbstoff angefärbt, der die Eigenschaft hat, die Zellmembran intakter Zellen nicht durchdringen zu können. Geschädigte oder sterbende Zellen hingegen erlauben das Eindringen des Farbstoffs und können so erfolgreich angefärbt werden. Zur Bestimmung der Anteile intakter und geschädigter Zellen wird eine stark verdünnte Zellsuspension in eine so genannte Neubauer-Zählkammer gegeben. Diese entspricht in ihren Dimensionen in etwa einem herkömmlichen Mikroskop-Objektträger und weist eine in eine Mehrzahl von Zählfeldern unterteilte Vertiefung definierter Größe auf. Unter dem Mikroskop können die Zellzahlen angefärbter, d.h. geschädigter, und nicht angefärbter, d.h. intakter, Zellen ausgezählt werden. Zur Verringerung des statistischen Fehlers werden üblicherweise die Zellzahlen in den einzelnen Zählfeldern bestimmt und gemittelt. Durch Rückrechnung mit den bekannten Werten des Zählkammervolumens und der gewählten Suspensionsverdünnung, können dann Aussagen über die absoluten Mengen intakter und geschädigter Zellen in der Zellkultur getroffen werden. Dieses Verfahren ist zum einen sehr aufwendig, da es eine Vielzahl manueller Schritte enthält. Zum anderen ist auch seine Genauigkeit stark limitiert. Insbesondere sind die auszählbaren Zellzahlen sehr beschränkt, was zu einem hohen statistischen Fehler führt. Nahezu untauglich ist das Verfahren daher, wenn nicht nur eine Aussage über die Anteile intakter und geschädigter Zellen, sondern zusätzlich eine differenzierte Aussage über die Verteilung des Schädigungsgrades der Zellen getroffen werden soll. Grundsätzlich wäre eine solche Aussage auf Basis einer Typhanblau-Anfärbung möglich, da der Grad der Anfärbung einer Zelle als Maß für deren Schädigung, insbesondere als Maß für den Fortschritt ihres Sterbeprozesses dienen kann. Um eine solche Verteilung jedoch statistisch zu validieren, müssten sehr große Mengen an Zellen ausgezählt werden, was das bekannte Verfahren vollständig ineffizient machen würde.

Aus dem Bereich der Diagnostik ist das so genannte PCV-Verfahren bekannt. PCV steht für "packed cell volume" und beschreibt den Anteil an Feststoffen in Form eines verdichteten Zellkuchens an dem Gesamtvolumen einer Probe. Weithin bekannt ist der als Hämatokrit bezeichnete PCV-Wert des Blutes. Zu seiner Bestimmung wird eine Blutprobe in einem meist zylindrischen Probenröhrchen zentrifugiert bis sich der Festbestandteil des Blutes als verdichteter Zellkuchen am Röhrchenboden abgesetzt hat. Der auch als Überstand bezeichnete Flüssiganteil schwimmt auf dem Zellkuchen. Zur Bestimmung des Hämatokrit wird das Volumen des Zellkuchens bestimmt und in ein prozentuales Verhältnis zum Gesamtvolumen der Probe gesetzt. Nachteilig bei der Bestimmung des PCV-Wertes ist, dass er lediglich einen Gesamtanteil an Feststoffen in einer Probe zulässt und insbesondere keine Aussage über die Viabilität der Zellen in diesem Feststoffanteil zulässt.

Es ist die Aufgabe der vorliegenden Erfindung, das bekannte Verfahren zur Viabilitätsbestimmung von Zellen derart zu verbessern, dass auf effiziente Weise eine genauere Bestimmung der Viabilität ermöglicht wird.

Diese Aufgabe wird in Verbindung mit den Merkmalen von Anspruch 1 dadurch gelöst, dass eine Zellsuspension der angefärbten Zellen als Probe in einem Probengefäß zentrifugiert wird, bis sich der in der Probe vorhandene Feststoffanteil als verdichteter Zellkuchen abgesetzt hat, und dass zur Anteilsbestimmung die Volumina unterschiedlich angefärbter Abschnitte des verdichteten Zellkuchens als Maß für die Anteile von Zellen unterschiedlicher Viabilität bestimmt werden. Dabei weist das Probengefäß einen Aufnahmebereich und einen sich an diesen anschließenden, als Kapillare ausgebildeten Messbereich auf, dessen lichte Weite wesentlich kleiner als die lichte Weite des Aufnahmebereichs ist. Hierdurch wird eine Maßstabstransformation erreicht, sodass ein gegebenes Volumen einer sehr viel größeren Höhe im Messbereich als im Aufnahmebereich entspricht.

Dies bedeutet, dass man die angefärbten Zellen den technischen Schritten des PCV-Verfahrens unterwirft, im Unterschied zum PCV-Verfahren jedoch nicht (nur) das Gesamtvolumen des verdichteten Zellkuchens, sondern die Teilvolumina seiner unterschiedlich angefärbten Abschnitte bestimmt.

Die Volumenbestimmung erfolgt vorzugsweise durch Ermittlung der Höhen der entsprechenden Zellkuchenabschnitte aus denen bei Kenntnis der Geometrie des Probengefäßes das entsprechende Volumen ermittelt werden kann. Insbesondere bei Verwendung von Probengefäßen mit gleich bleibendem Querschnitt im Bereich des Zellkuchens entspricht die Höhenverteilung der einzelnen Abschnitte direkt der Volumenverteilung.

Wesentliche Grundlage der Erfindung ist die Erkenntnis, dass die von intakten und geschädigten Zellen herrührenden Feststoffanteile eine unterschiedliche Dichte aufweisen, so dass sie durch Zentrifugieren trennbar sind. Überraschenderweise hat sich gezeigt, dass dies nicht nur für vollständig intakte Zellen einerseits und zerstörte Zellen, d.h. Zellfragmente, andererseits gilt, sondern dass sich auch angefärbte Zellen in unterschiedlichen Stadien ihres Sterbeprozesses hinsichtlich ihrer Dichte unterscheiden und somit durch Zentrifugieren trennbar sind.

Dies kann in einer besonders vorteilhaften Ausführungsform des erfindungsgemäßen Verfahrens dadurch ausgenutzt werden, dass zur Bestimmung der Verteilung von Zellen unterschiedlichen Schädigungsgrades ein Anfärbungsverlauf in Richtung der Zellkuchenhöhe wenigstens eines Abschnitts des Zellkuchens ermittelt wird. In der Regel wird man bei typischen Zellkulturen drei unterscheidbare Abschnitte des Zellkuchens finden. In einem ersten Bereich, in dem tote Zellen und Zellfragmente abgelagert sind, wird eine einheitliche, maximale Anfärbung vorgefunden werden. In einem anderen Bereich, der vollständig intakte Zellen erreicht, wird praktisch keine Anfärbung vorgefunden werden. Dazwischen wird sich in der Regel ein Übergangsbereich erstrecken, der in geordneter Weise unterschiedliche Schattierungen von fast minimaler bis fast maximaler Anfärbung aufweisen wird. Dieser Bereich zeigt einen Anfärbungsverlauf oder Anfärbungsgradienten in Richtung der Zellkuchenhöhe. Eine detaillierte Bestimmung dieses Anfärbungsverlaufs repräsentiert die Verteilung unterschiedlich geschädigter Zellen. Eine solche differenzierte Betrachtung ist dank der vorliegenden Erfindung sinnvoll, da im Gegensatz zu den Viabilitäts-Bestimmungsverfahren nach dem Stand der Technik eine große Anzahl von Zellen zu dem messbaren Anfärbungssignal beitragen, so dass die genannte Verlaufsbestimmung auch statistisch valide ist.

Während sich bei dem oben beschriebenen Hämatokrit-Messverfahren die typischen Messwerte im Bereich von 30-50% bewegen, sind die bei Zellkulturen zu erwartenden Gesamtfeststoffwerte deutlich geringer, typischerweise im Bereich von 1% oder darunter. Dies bedeutet, dass bei Verwendung der für die Hämatokritbestimmung üblichen Probengefäße für das erfindungsgemäße Verfahren die Höhe der Überstandssäule etwa ein Hundertfaches der Höhe des Zellkuchens beträgt. Dies kann leicht zu großen Ablesefehlern führen, was sich insbesondere nachteilig auswirkt, wenn, wie oben diskutiert, detailliertere Aussagen über die Verteilung unterschiedlich viabler Zellen getroffen werden sollen.

Als besonders praxistauglich hat sich die Verwendung einer Kapillare mit Durchmesser im Bereich von 500 Mikrometern und einem Volumen von einem bis zu zehn Mikrolitern, insbesondere zwei bis sieben Mikrolitern und besonders bevorzugt von etwa fünf Mikrolitern als Messbereich erwiesen. Der Aufnahmebereich, dessen Durchmesser in der Größenordnung von einigen Millimetern bis zu einigen Zentimetern betragen kann, weist vorzugsweise ein Volumen in der Größenordnung von Millilitern, insbesondere zwei Milliliter oder weniger, bevorzugt etwa ein Milliliter, auf. Diese Dimensionierung führt dazu, dass sich bei der Untersuchung typischer Zellkulturen ein verdichteter Zellkuchen nur im Messbereich ausbildet, wohingegen der Aufnahmebereich nach der Zentrifugation ausschließlich mit Überstand gefüllt ist.

Die Maßstabstransformation führt zum einen zu der Möglichkeit eines sehr genauen Ablesens der Höhen der einzelnen Zellkuchenabschnitte, da bereits kleine Volumenänderungen zu vergleichsweise großen und gut ablesbaren Höhenänderungen des entsprechenden Zellkuchenabschnitts führen. Andererseits erleichtert diese Ausführungsform die Bestimmung des Anfärbungsverlaufs oder -gradienten zur Ermittlung der Verteilung unterschiedlich geschädigter Zellen, da die optische Dichte des Zellkuchens senkrecht zu seiner Höhe sehr gering ist. Bei Anfärbung mit einem lichtabsorbierenden Farbstoff, wie z.B. Tryphanblau, können sehr genaue Anfärbungswerte, insbesondere auch im Bereich starker Anfärbung, in Transmission gemessen werden.

Alternativ können auch fluoreszierende oder phosphoreszierende, allgemeiner ausgedrückt als lichtemittierende, Farbstoffe verwendet werden. Bei dieser Ausführungsform des erfindungsgemäßen Verfahrens ist zur Beleuchtung bei der Auswertung eine übliche Fluoreszenzmessanordnung zu verwenden. Die geringe optische Dichte des Zellkuchens senkrecht zu seiner Höhe hat dabei den Vorteil, dass Artefakte durch Streuung oder Reabsorption weitestgehend vermieden werden können.

Bei einer vorteilhaften Weiterbildung des erfindungsgemäßen Verfahrens ist vorgesehen, dass ein bildgebender Sensor den Zellkuchen abbildet und ein digitales Bild an eine Datenverarbeitungseinrichtung leitet, die das digitale Bild nach vorgegebenen Regeln zur Bestimmung der Höhen der Zellkuchenabschnitte analysiert. Dies erlaubt eine automatisierte Auswertung. Es sind dem Fachmann Bilderkennungsalgorithmen bekannt, die in automatisierter Weise den abgebildeten Zellkuchen hinsichtlich seiner Höhe und der Verteilung seiner Anfärbung vermessen können. Der Begriff des bildgebenden Sensors ist dabei weit zu verstehen und umfasst sowohl einen Flächensensor, wie z.B. einen CCD-Sensor, als auch einen geeignet zum Zellkuchen ausgerichteten Linearsensor oder eine den Zellkuchen abrasternde Sensoreinrichtung.

Wie erwähnt, spielt die Tatsache, dass sich Zellen unterschiedlicher Viabilität in ihrer Dichte unterscheiden, eine für die vorliegende Erfindung wichtige Rolle. Dies führt andererseits jedoch dazu, dass die relativen Höhen einzelner Zellkuchenabschnitte nicht unmittelbar die relative Volumenverteilung der entsprechenden Zellen in der nicht als Zellkuchen verdichteten Probe wiedergeben. Entsprechend ist bei einer bevorzugten Ausführungsform des erfindungsgemäßen Verfahrens vorgesehen, dass zur Berechnung der Anteile von Zellen unterschiedlicher Viabilität die ermittelten Höhen der Zellkuchenabschnitte mit Wichtungsparametern gewichtet werden, die unterschiedliche Dichten von Zellmaterial unterschiedlicher Viabilität repräsentieren. Somit kann der Einfluss unterschiedlicher Verdichtung der einzelnen Abschnitte im Zellkuchen korrigiert werden. Dies erfolgt vorzugsweise automatisiert durch die Datenverarbeitungseinrichtung.

Für die Durchführung des Zentrifugationsschrittes haben sich relative Zentrifugalkräfte von 2000 bis 3000 g, insbesondere bei 2250 bis 2750 g, insbesondere bei etwa 2500 g als gut praktikabel erwiesen. Als günstige Zentrifugationszeiten haben sich Perioden von 0,5 bis 5 Minuten, insbesondere 0,5 bis 2 Minuten, insbesondere etwa 1 Minute erwiesen. Als optimale Standardwerte für den Zentrifugationsschritt hat sich eine Zentrifugation über etwa eine Minute bei relativen Zentrifugalkräften von etwa 2500 g erwiesen. "Optimal" bedeutet dabei den bestmöglichen Kompromiss aus Verfahrenseffizienz, d.h. insbesondere Verfahrensdauer, hinreichender Verdichtung des Zellkuchens, Reproduzierbarkeit des Ergebnisses und Maßhaltigkeit des Ergebnisses nach Zentrifugation.

Weitere Merkmale und Vorteile der Erfindung ergeben sich aus der nachfolgenden, speziellen Beschreibung sowie den Zeichnungen.

Es zeigen:
- Figur 1:: eine schematische Darstellung einer bevorzugten Ausführungsform eines Probengefäßes;
- Figur 2:: eine um 90° um die Symmetrieachse rotierte Ansicht eines Probengefäßes gemäß Figur 1;
- Figur 3:: eine vergrößerte Darstellung des Messbereichs eines Probengefäßes gemäß Figur 1;
- Figur 4:: drei Probengefäße gemäß Figur 1 in einer bevorzugten Ausführungsform eines Probenhalters;
- Figur 5:: eine erste perspektivische Darstellung einer Ausführungsform eines Auslesegerätes;
- Figur 6:: eine zweite perspektivische Darstellung des Auslesegerätes nach Figur 4; und
- Figur 7:: eine schematische Darstellung einer Ergebniskurve als Resultat des erfindungsgemäßen Verfahrens.

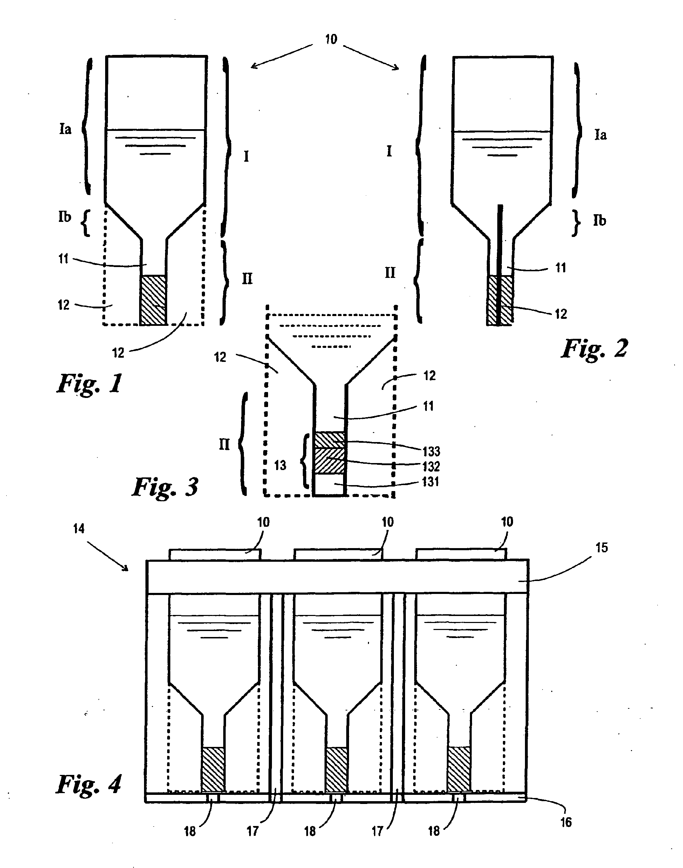

Figuren 1 und 2 zeigen eine schematische Darstellung einer besonders bevorzugten Ausführungsform eines Probengefäßes, nämlich eines so genannten PCV-Röhrchen 10, zur Durchführung des erfindungsgemäßen Verfahrens. Das PCV-Röhrchen 10 kann in zwei Hauptbereiche unterteilt werden. Ein erster, großvolumiger Bereich I wird hier als Aufnahmebereich I bezeichnet. Ein zweiter, als Kapillare ausgebildeter Bereich II wird hier als Messbereich bezeichnet. Der Aufnahmebereich ist bei der in Figuren 1 und 2 dargestellten Ausführungsform in zwei Unterbereiche unterteilt: einen im Wesentlichen zylindrischen Hauptbereich Ia und einen keglig zulaufenden Übergangsbereich Ib. Typische Abmessungen für den Aufnahmebereich I liegen in der Größenordnung von cm, sein typisches Volumen in der Größenordnung von etwa 1 ml. Der Messbereich II ist als Kapillare mit einer typischen lichten Weite von etwa 500 µm ausgebildet. Sein typisches Volumen liegt bei etwa 5 µl. Es sei ausdrücklich darauf hingewiesen, dass diese Maßangaben lediglich in der Praxis bewährte Beispielmaße darstellen. Diese können, ohne sich vom Kern der vorliegenden Erfindung zu entfernen, vom Fachmann in Anbetracht der konkreten Anwendung angepasst werden. Insbesondere in Fällen, in denen hohe PCV-Werte zu erwarten sind, kann das Volumen des Messbereichs II größer gestaltet werden. In Fällen, in denen Kulturen besonders großer Zellen untersucht werden sollen, kann beispielsweise die lichte Weite der Kapillare des Messbereichs II entsprechend groß gestaltet werden. Umgekehrtes gilt selbstverständlich analog für Fälle besonders kleiner Zellen und/oder geringer erwarteter PCV-Werte.

Das PCV-Röhrchen ist wenigstens in seinem Messbereich II aus transparentem Material, vorzugsweise aus transparentem Kunststoff gefertigt. Bei der in Figuren 1 und 2 dargestellten, besonders vorteilhaften Ausführungsform sind angrenzend an den Messbereich II und den Übergangsbereich Ib zwei Stabilisierungsflügel 12 vorgesehen. Die Stabilisierungsflügel 12 sind vorzugsweise einstückig mit den Außenwänden des Messbereichs II und des Übergangsbereichs Ib ausgebildet und verlaufen so, dass wenigstens bei einer gegebenen Orientierung des PCV-Röhrchens 10 ein unverstellter Blick auf den Messbereich II möglich ist. Obgleich bei der in den Figuren 1 und 2 dargestellten Ausführungsform zwei Stabilisierungsflügel 12 dargestellt sind, ist ihre Anzahl grundsätzlich nicht beschränkt. Auch könnte es sich anstelle von Flügeln 12 um einen oder mehrere Stabilisierungskörper, beispielsweise in zylindrischer Fortsetzung des Hauptsaufnahmebereichs Ia handeln. Wichtig ist jedoch die unverstellte Einsichtmöglichkeit in wenigstens einer Orientierung des PCV-Röhrchens 10.

Zur Durchführung des erfindungsgemäßen Verfahrens wird eine für eine zu untersuchende Zellkultur repräsentative Zellsuspension mit einem Viabilitätsfarbstoff angefärbt. Wie bereits erwähnt, stellt Tryphanblau einen geeigneten solchen Farbstoff dar. Dem Fachmann sind jedoch weitere derartige Farbstoffe bekannt, die in Abhängigkeit von der Viabilität einzelner Zellen diese mehr oder weniger stark anfärben. Hierunter fallen sowohl absorbierende als auch lichtemittierende, insbesondere fluoreszierende oder phosphoreszierende Farbstoffe.

Nach hinreichender Inkubationszeit, d.h. nach einer Einwirkzeit des Farbstoffs auf die Zellsuspension, die für eine viabilitätsabhängige Anfärbung der zu untersuchenden Zellen angemessen ist, wird die Zellsuspension in dem PCV-Röhrchen 10 zentrifugiert. Als besonders günstig hat sich eine Zentrifugation bei etwa 2500 g über ca. 1 min ergeben. Deutlich geringere relative Zentrifugalkräfte und/oder kürzere Zentrifugationszeiten führen zu einer nicht ausreichenden Verdichtung des Zellkuchens. Bei deutlich größeren relativen Zentrifugalkräften beobachtet man dagegen eine Reexpansion des Zellkuchens nach Abschluss des Zentrifugationsschrittes. Dies führt zu unerwünschten Messungenauigkeiten. Deutlich längere Zentrifugationszeiten führen zu keiner nennenswerten Verdichtungsverbesserung oder Reproduzierbarkeit des Ergebnisses und sind daher aufgrund der unmittelbar mit ihnen verbundenen Verlängerung der Verfahrensdauer nicht sinnvoll. Es sei allerdings erwähnt, dass besondere Zelltypen zur Ergebnisoptimierung andere Zentrifugationsparameter erfordern. Eine entsprechende Auswahl zu treffen, liegt im Bereich des durchschnittlichen Fachmannkönnens.

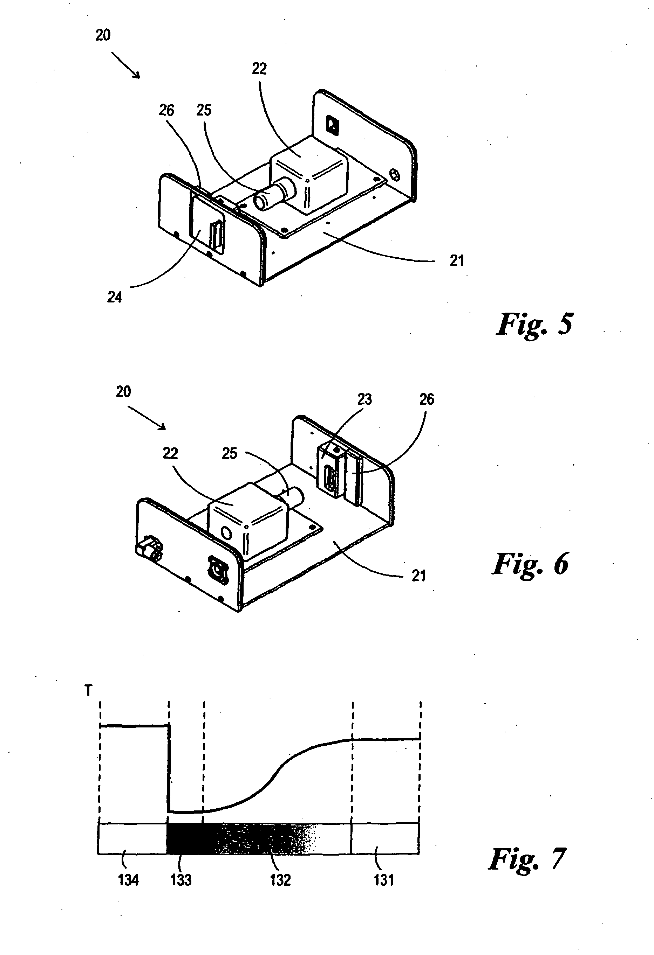

Figur 3 zeigt den Messbereich II eines PCV-Röhrchen 10 in vergrößerter, schematischer Darstellung. Im unteren Bereich der Kapillare hat sich ein Zellkuchen 13 abgesetzt. Der Zellkuchen 13 ist in drei Abschnitte unterschiedlicher Höhen unterteilt. In einem ersten Zellkuchenabschnitt 131, der sich aufgrund der hohen Dichte des ihn bildenden Materials im unteren Bereich der Kapillare 11 absetzt, befinden sich die vollständig intakten Zellen. Diese sind im gezeigten Ausführungsbeispiel, nicht angefärbt, da ihre Zellmembranen einem Eindringen des Viabilitätsfarbstoffes widerstanden haben. In einem weiteren Bereich 133, der aufgrund der besonders niedrigen Dichte des ihn bildenden Materials den obersten Bereich des Zellkuchens 13 bildet, sind abgestorbene Zellen und Zellfragmente enthalten. Hier konnten die Zellmembranen einem Eindringen des Viabiliätsfarbstoffes nichts entgegen setzen, so dass der Bereich 133 sich durch maximale Anfärbung auszeichnet. Zwischen den Bereichen 131 und 133 erstreckt sich ein Bereich 132, der diejenigen Zellen enthält, deren Viabilitätsgrad zwischen vollständig intakt und vollständig abgestorben liegt. In diesem Bereich wird sich ein Farbgradient ausbilden, wie er schematisch in Figur 7, auf die später eingegangen werden soll, dargestellt ist.

Figur 4 zeigt einen vorteilhaften Röhrchenhalter zur Aufnahme von einem oder mehreren PCV-Röhrchen 10. Der Halter 14 besteht im Wesentlichen aus einer Deckplatte 15 mit Ausnehmungen, deren Durchmesser im Wesentlichen dem Außendurchmesser der PCV-Röhrchen 10 entspricht, sowie einer Bodenplatte 16, auf der die PCV-Röhrchen 10 aufstehen. Verbunden sind die Deckplatte 15 und die Bodenplatte 16 durch Separationswände 17, die bei einer bevorzugten Ausführungsform der Erfindung eine optische Isolierung zwischen den einzelnen PCV-Röhrchen darstellen. Die Vorder- und/oder Rückseite des Halters bleiben vorzugsweise frei, um eine flächige Beleuchtung des PCV-Röhrchens und eine Beobachtung des Transmissionslichtes zu ermöglichen. Bei der dargstellten Ausführungsform sind zusätzliche Öffnungen 18 in der Bodenplatte des Haltes vorgesehen, die auch eine Beleuchtung parallel zur Erstreckungsrichtung der PCV-Röhrchen 10 erlauben; dies ist insbesondere bei Verwendung eines Fluoreszenz-Viabilitätfarbstoffes günstig.

Der Halter 14 kann eine beliebige Anzahl von Kammern zur Aufnahme von PCV-Röhrchen 10 umfassen. Die einzelnen Kammern sind vorzugsweise so dimensioniert, dass auch herkömmliche Zentrifugationsröhrchen, an deren Dimensionen die bevorzugten PCV-Röhrchen 10 angepasst sind, darin abgestellt werden können. Bei einer besonders günstigen Ausführungsform des Halters 14 ist dieser so gestaltet, dass er in handelsübliche Zentrifugen eingesetzt werden kann. Auf diese Weise wird es ermöglicht, mehrere PCV-Röhrchen 10 gleichzeitig in eine Zentrifuge einzustellen oder zu entnehmen.

Figuren 5 und 6 zeigen eine bevorzugte Ausführungsform eines Lesegerätes 20 zur Auswertung des mit dem erfindungsgemäßen Verfahren ermittelten Messergebnisses. Das Gerät umfasst ein vorzugsweise lichtdichtes Gehäuse 21, von dem in den Figuren 5 und 6 lediglich ein unterer Trägerteil dargestellt ist. Ein vorzugsweise vorgesehener korrespondierender Deckel ist in den Figuren 5 und 6 nicht gezeigt. Das Lesegerät 20 umfasst weiter einen bildgebenden Sensor 22, der vorzugsweise als flächige CCD-Kamera ausgestaltet ist. Alternativ können auch Zeilensensoren oder rasternde Fotosensoren vorgesehen sein. Weiter umfasst das Lesegerät 20 eine Probenhalterung 23, die vorzugsweise an der Innenseite einer schwenkbaren Tür 24 in einer Stirnseite des Gerätes 20 angeordnet ist. Die Haltevorrichtung 23 ist vorzugsweise auf die Dimensionen der PCV-Röhrchen 10 abgestimmt und beispielsweise unter Verwendung von Führungen und/oder Federklemmungen so gestaltet, dass eingesetzte Probengefäße stets dieselbe Positionierung und Ausrichtung zeigen. Bei der in den Figuren 5 und 6 dargestellten, bevorzugten Ausführungsform kann ein Probengefäß bei geöffneter Tür 24 leicht in den Probenhalter 23 eingesetzt werden. Nachfolgendes Schließen der Tür 24 positioniert dasProbengefäß in Gegenüberstellung zum Sensor 22 und verschließt gleichzeitig das Gehäuse 21 lichtdicht.

Je nach spezieller Ausgestaltung des Sensors 22 und seiner relativen Orientierung zum Probenhalter 23 ist eine Abbildungsoptik 25 vorgesehen, die wenigstens den Bereich des verdichteten Zellkuchens 13 in dem Probengefäß auf den Sensor 22 abbildet. Zur Abbildung auf den Sensor 22 wird die Probe im Probenhalter 23 von einer Beleuchtungsanordnung 26, die bei der dargestellten Ausführungsform benachbart zu dem Probenhalter 23 angeordnet ist, beleuchtet. Die Beleuchtung erfolgt vorzugsweise durch eine Anordnung einer oder mehreren Leuchtdioden, deren Licht vorzugsweise so in den Probenhalter eingekoppelt wird, dass die Probe, insbesondere der Messbereich II eines PCV-Röhrchens 10 gleichmäßig von hinten durchstrahlt wird. Alternativ oder zusätzlich können andere Einkopplungsmöglichkeiten für andere oder weitere Lichtquellen gegeben sein. Beispielsweise können externe Lichtquellen, wie beispielsweise externe Laser, über Spiegel oder Glasfasern eingekoppelt werden. Das Vorsehen verschiedener Einkopplungsmöglichkeiten, insbesondere unter Verwendung von Standardkomponenten, ermöglicht einen einfachen und variablen Einsatz des Gerätes 20 für unterschiedliche Viabilitätsfarbstoffe und Beleuchtungserfordernisse. Alternativ zu dem dargestellten Probenhalter 23 kann auch eine Mehrzahl von Probenhaltern vorgesehen sein, insbesondere solche, die zur Aufnahme der zuvor beschriebenen Halter 14 geeignet sind, wobei günstiger Weise ein manueller oder motorischer Antrieb vorgesehen ist, der einen automatisierten Probenvorschub und eine automatisierte Probenauswertung erlaubt.

Der Sensor 22 ist vorzugsweise mit einer digitalen Datenverarbeitungseinrichtung gekoppelt. Dabei kann es sich um einen externen Rechner oder auch um einen Mikroprozessor handeln, der in dem Gehäuse 21 angeordnet ist. Die Datenverarbeitungseinrichtung nimmt die von dem Sensor erzeugten, digitalen Bilder auf und führt sie einem Auswertungsprozess nach vorgegebenen Regeln zu. Ziel der Auswertung ist es, die Höhe des Zellkuchens und insbesondere der einzelnen Zellkuchenabschnitte im Probengefäß zu bestimmen. Hierzu sind dem Fachmann geeignete Bildverarbeitungsalgorithmen bekannt.

Bei einer besonders bevorzugten Ausführungsform des erfindungsgemäßen Verfahrens wird zusätzlich der Farbverlauf im mittleren Zellkuchenabschnitt 132 detailliert analysiert. Eine schematische Kurve ist in Figur 7 dargestellt. Im unteren Bereich von Figur 7 ist schematisch ein Messbereich eines PCV-Röhrchens 10 dargestellt. Deutlich erkennbar sind die unterschiedlichen Zellkuchenabschnitte, nämlich der Abschnitt 131 mit vollständig intakten Zellen, der Bereich 133 mit vollständig abgestorbenen Zellen und Zellfragmenten sowie der mittlere Bereich 132 mit Zellen unterschiedlichen Schädigungsgrades. Zusätzlich erkennbar in Figur 7 ist ein weiterer Bereich 134, der wie der Bereich 131 nicht angefärbt ist. Hierbei handelt es sich um einen "Leerabschnitt" des Messbereichs, der bei dem gezeigten Beispiel nicht vollständig vom Zellkuchen ausgefüllt ist. In diesem Abschnitt 134 sammelt sich Überstand, d.h. feststofffreie Probenlösung. Diese ist nicht angefärbt.

Im oberen Bereich von Figur 7 ist schematisch eine Transmissionskurve dargestellt, wie sie sich etwa aus der Analyse des dargestellten Messbereichs ergeben könnte. Der Abschnitt 134 zeigt, da zellfrei, eine sehr hohe Transmission. Im Gegensatz dazu zeigt der Abschnitt 133 maximale Färbung, d.h. minimale Transmission. Der Übergang zwischen den Abschnitten 134 und 133 ist als Phasengrenze unstetig. Im Abschnitt 133 ist die Transmission im Wesentlichen konstant. Im entfernt gelegenen Abschnitt 131 ist die Transmission ebenfalls konstant, liegt jedoch auf einem deutlich höheren Niveau. Bei dem Beispiel von Figur 7 liegt dieses Niveau etwas unterhalb des Transmissionsnivaus in Abschnitt 134, da hier aufgrund der dichten Zellpackung mit Lichtstreuung und damit geringfügig verringerter Transmission gegenüber dem reinen Überstand in Abschnitt 134 zu rechnen ist. Im Zwischenabschnitt 132 hingegen ergibt sich ein stetiger Farbverlauf zwischen den Abschnitten 133 und 131. Eine Analyse dieses Bereiches kann eine differenzierte Aussage über den "Gesundheitszustand" der untersuchten Zellkultur erlauben, die über eine bloße quantitative Bestimmung des Gesamtzellvolumens und der Volumina abgestorbener und intakter Zellen hinausgeht. Ein ähnliches bzw. analoges Schema gilt selbstverständlich auch für eine Messung in einem Reflexions- oder Emissionsmodus. Hier wird der Fachmann die Beleuchtung entsprechend den speziellen optischen Anforderungen der Messung anpassen und das als Messergebnis resultierende Bild angemessen interpretieren bzw. automatisiert von einer entsprechenden Vorrichtung auswerten lassen.

Natürlich stellen die in der speziellen Beschreibung diskutierten und in den Figuren gezeigten Ausführungsformen lediglich illustrative Ausführungsbeispiele der vorliegenden Erfindung dar. Dem Fachmann steht ein weites Spektrum an Modifikationsmöglichkeiten zur Verfügung. Insbesondere ist die Wahl der Zellen, der Farbstoffe, der Gefäßformen und -dimensionen, der Beleuchtungsanordnung und der Auslesevorrichtung der Anpassung durch den Fachmann an jeweils konkreten Aufgabenstellungen und Anwendungsbedingungen unterworfen.

## Patentansprüche

1. Verfahren zum Bestimmen der Viabilität von Zellen in Zellkulturen, umfassend die Schritte:
- Anfärben der Probe mit einem Farbstoff, der in Abhängigkeit von der Viabilität einzelner Zellen in diese einzudringen vermag,
- Bestimmen der Anteile unterschiedlich stark angefärbter Zellen,
**dadurch gekennzeichnet,**
**dass** eine Zellsuspension der angefärbten Zellen als Probe in einem Probengefäß (10) zentrifugiert wird, bis sich der in der Probe vorhandene Feststoffanteil als verdichteter Zellkuchen (13) abgesetzt hat, und dass zur Anteilsbestimmung die Volumina unterschiedlich angefärbter Abschnitte (131, 132, 133) des verdichteten Zellkuchens (13) als Maß für die Anteile von Zellen unterschiedlicher Viabilität bestimmt werden, wobei das Probengefäß (10) einen Aufnahmebereich (I) und einen sich an diesen anschließenden, als Kapillare ausgebildeten Messbereich (II) aufweist, dessen lichte Weite wesentlich kleiner als die lichte Weite des Aufnahmebereichs (I) ist.

2. Verfahren nach Anspruch 1,
**dadurch gekennzeichnet,**
**dass** die Volumenbestimmungen durch Ermittlung der Höhen der entsprechenden Zellkuchenabschnitte (131, 132, 133) vorgenommen werden.

3. Verfahren nach Anspruch 2,
**dadurch gekennzeichnet,**
**dass** zur Bestimmung der Verteilung von Zellen unterschiedlichen Schädigungsgrades ein Anfärbungsverlauf in Richtung der Zellkuchenhöhe wenigstens eines Abschnitts (132) des Zellkuchens (13) ermittelt wird.

4. Verfahren nach einem der Ansprüche 2 bis 3,
**dadurch gekennzeichnet,**
**dass** ein bildgebender Sensor (22) den Zellkuchen (13) abbildet und ein digitales Bild an eine Datenverarbeitungseinrichtung leitet, die das digitale Bild nach vorgegebenen Regeln zur Bestimmung der Höhen der Zellkuchenabschnitte (131, 132, 133) analysiert.

5. Verfahren nach einem der Ansprüche 2 bis 4,
**dadurch gekennzeichnet,**
**dass** zur Berechnung der Anteile von Zellen unterschiedlicher Viabilität die ermittelten Höhen der Zellkuchenabschnitte (131, 132, 133) mit Wichtungsparametern gewichtet werden, die unterschiedliche Dichten von Zellmaterial unterschiedlicher Viabilität repräsentieren.

6. Verfahren nach einem der vorangehenden Ansprüche,
**dadurch gekennzeichnet,**
**dass** der Farbstoff ein Licht absorbierender und/oder streuender Farbstoff, insbesondere Tryphanblau ist.

7. Verfahren nach einem der Ansprüche 1 bis 5,
**dadurch gekennzeichnet,**
**dass** der Farbstoff ein Licht emittierender Farbstoff ist.

8. Verfahren nach einem der vorangehenden Ansprüche,
**dadurch gekennzeichnet,**
**dass** die Zentrifugation bei relativen Zentrifugalkräften von 2000 bis 3000 g, insbesondere bei 2250 bis 2750 g, insbesondere bei etwa 2500 g erfolgt.

9. Verfahren nach einem der vorangehenden Ansprüche,
**dadurch gekennzeichnet,**
**dass** der Zentrifugationsschritt 0,5 bis 5 min, insbesondere 0,5 bis 2 min, insbesondere etwa 1 min dauert.

## Claims

1. Method of determining the viability of cells in cell cultures, comprising the steps:
- colouring the sample with a dye which is capable of penetrating into individual cells depending on the viability thereof and
- determining the proportions of cells coloured to different extent,
**characterised in that** a cell suspension of the coloured cells as sample is centrifuged in a sample vessel (10) until the solid component present in the sample has sedimented as compacted cake (13) and that for the proportion determination the volumes of differently coloured sections (131, 132, 133) of the compacted cell cake (13) are determined as a measure for the proportions of cells of different viability, wherein the sample vessel (10) has a receiving region (I) and a measuring region (II), which is connected therewith and is constructed as a capillary and the clear width of which is substantially smaller than the clear width of the receiving region (I).

2. Method according to claim 1, **characterised in that** the volume determinations are carried out by ascertaining the heights of the corresponding cell cake sections (131, 312, 133).

3. Method according to claim 2, **characterised in that** for determining the distribution of cells of different degree of deterioration a coloration plot is ascertained of at least a section (132) of the cell cake (13) in the direction of the cell cake height.

4. Method according to one of claims 2 and 3, **characterised in that** an image-transmitting sensor (22) images the cell cake (13) and conducts a digital image to a data processing device, which analyses the digital image in accordance with predetermined rules for determination of the heights of the cell cake sections (131, 132, 133).

5. Method according to any one of claims 2 to 4, **characterised in that** for calculation of the proportions of cells of different viability the ascertained heights of the cell cake sections (131, 132, 133) are weighted by weighting parameters representing different densities of cell material of different viability.

6. Method according to any one of the preceding claims, **characterised in that** the dye is a light-absorbing and/or dispersing dye, particularly trypan blue.

7. Method according to any one of claims 1 to 5, **characterised in that** the dye is a light-emitting dye.

8. Method according to any one of the preceding claims, **characterised in that** the centrifuging is carried out at relative centrifugal forces of 2000 to 3000 g, particularly at 2250 to 2750 g, especially at approximately 2500 g.

9. Method according to any one of the preceding claims, **characterised in that** the centrifuging step lasts for 0.5 to 5 minutes, particularly 0.5 to 2 minutes, especially approximately 1 minute.

## Revendications

1. Procédé de détermination de la viabilité des cellules dans les cultures de cellules, comprenant les étapes suivantes :
- coloration de l'échantillon avec un colorant qui peut pénétrer dans celui-ci en fonction de la viabilité des différentes cellules,
- détermination des proportions de cellules très différemment colorées,
**caractérisé en ce qu'**une suspension cellulaire de cellules colorées est centrifugée dans un récipient d'échantillons (10) en tant gu'échantillon, jusqu'à ce que la proportion de matières solides contenues dans l'échantillon se soit déposée sous forme de gâteau de cellules consolidé (13), et **en ce que**,
pour déterminer la proportion, on détermine les volumes de segments différemment colorés (131, 132, 133) du gâteau de cellules consolidé (13) comme mesure pour les proportions de cellules qui présentent une viabilité différente, sachant que le récipient d'échantillons (10) présente un segment de réception (I) et une zone de mesure (II) en forme de vaisseau capillaire se raccordant à celui-ci dont le diamètre intérieur est sensiblement plus petit que le diamètre intérieur du segment de réception (I).

2. Procédé selon la revendication 1, **caractérisé en ce qu'**on procède aux déterminations des volumes en recherchant les hauteurs correspondantes des sections du gâteau de cellules (131, 132, 133).

3. Procédé selon la revendication 2, **caractérisé en ce que,** pour déterminer la répartition des cellules qui présentent un degré de détérioration différent, on recherche une variation de la coloration en direction de la hauteur du gâteau de cellules au moins d'un segment (132) du gâteau de cellules (13).

4. Procédé selon l'une quelconque des revendications 2 à 3, **caractérisé en ce que,** pour déterminer les hauteurs des sections du gâteau de cellules (131, 132, 133), un capteur d'imagerie (22) reproduit le gâteau de cellules (13) et dirige une image numérique vers un dispositif de traitement des données qui analyse l'image numérique selon des règles fixées à l'avance.

5. Procédé selon l'une quelconque des revendications 2 à 4, **caractérisé en ce que,** pour calculer les proportions de cellules qui présentent une viabilité différente, on pondère les hauteurs déterminées des sections du gâteau de cellules (131, 132, 133) avec des paramètres de pondération qui représentent les différentes densités de matière cellulaire.

6. Procédé selon l'une quelconque des revendications précédentes, **caractérisé en ce que,** le colorant est un colorant qui absorbe et/ou diffuse de la lumière, en particulier le bleu trypan.

7. Procédé selon l'une quelconque des revendications 1 à 5, **caractérisé en ce que,** le colorant est un colorant qui émet de la lumière.

8. Procédé selon l'une quelconque des revendications précédentes, **caractérisé en ce que,** la centrifugation a lieu lorsque les forces centrifuges relatives sont comprises dans la plage allant de 2000 à 3000 g, en particulier de 2250 à 2750 g, en particulier d'environ 2500 g.

9. Procédé selon l'une quelconque des revendications précédentes, **caractérisé en ce que,** l'étape de centrifugation est d'une durée de 0,5 à 5 minutes, en particulier de 0,5 à 2 minutes, en particulier d'environ 1 minute.
